# EUROPÄISCHE PATENTSCHRIFT

(11) **EP 1 019 371 B1**
(45) Veröffentlichungstag und Bekanntmachung des Hinweises auf die Patenterteilung: **02.05.2003**
(21) Anmeldenummer: 96938012.0
(22) Anmeldetag: 11.09.1996
(51) Int. Cl.: C07D 207/44, C08J 3/24

(54) **ALS VERKNÜPFUNGSREAGENZIEN EINSETZBARE DIMALEINIMIDOSUBSTITUIERTE DIHYDROXYALKANE UND VERFAHREN ZU DEREN HERSTELLUNG**
DIMALEINIMIDO-SUBSTITUTED DIHYDROXYALKANES WHICH CAN BE USED AS CROSSLINKING REAGENTS AND PROCESS FOR THEIR PREPARATION
DIHYDROXYALCANES SUBSTITUES PAR DIMALEINIMIDO S'UTILISANT COMME REACTIFS DE RETICULATION ET PROCEDE PERMETTANT DE LES PREPARER

(30) Priorität: 13.09.1995 DE 19533867
(43) Veröffentlichungstag der Anmeldung: 19.07.2000
(73) Patentinhaber: Forschungszentrum Jülich GmbH, 52425 Jülich (DE); Forschungsverbund Berlin e.V., 12489 Berlin (DE)
(72) Erfinder: BAUER, Paul, J., D-52428 Jülich (DE); HAGEN, Volker, D-13051 Berlin (DE)
(86) Internationale Anmeldenummer: DE9601742
(87) Internationale Veröffentlichungsnummer: WO97010209

(56) Entgegenhaltungen:
- DE-A- 4 014 540
- JOURNAL OF THE AMERICAN CHEMICAL SOCIETY, Bd. 78, Nr. 11, 5.Juni 1956, WASHINGTON DC, US, Seiten 2414-8, XP000651240 J. E. MOORE ET. AL.: "Cross-linking of Bovine Plasma Albumin and Wood Keratin" in der Anmeldung erwähnt
- JOURNAL OF BIOLOGICAL CHEMISTRY, Bd. 263, Nr. 2, 15.Januar 1988, BALTIMORE, US, Seiten 938-44, XP000651223 P. D. CHANTLER ET. AL.: "Cross-linking between Translationally Equivalemt Sites on the Two Heads of Myosin" in der Anmeldung erwähnt
- JOURNAL OF THE AMERICAN CHEMICAL SOCIETY, Bd. 81, Nr. 5, 5.März 1959, WASHINGTON DC, US, Seiten 1187-9, XP000651249 P. KOVACIC ET. AL.: "Cross-linking of Polymers with Dimaleimides." in der Anmeldung erwähnt
- JOURNAL OF BIOCHEMISTRY, Bd. 90, Nr. 4, 1981, Seiten 1177-85, XP000651257 S. SATO ET. AL.: "Cross-linking of Intact Erythrocyte Membrane with a Newly Synthesised Cleavable Bifunctional Reagent" in der Anmeldung erwähnt

## Beschreibung

Die Erfindung betrifft neue dimaleinimidosubstituierte Dihydroxyalkane gemäß den Ansprüchen 1 und 2, ein Verfahren zu deren Herstellung gemäß den Ansprüchen 3 bis 6 sowie deren Verwendung als Verknüpfungsreagenzien gemäß Anspruch 7.

In der Proteinforschung ist insbesondere der Einsatz bifunktioneller Verknüpfungsreagenzien ("cross-linker") weit verbreitet. Es handelt sich dabei um Substanzen, die über zwei funktionelle Gruppen verfügen, die entweder identisch sind (homobifunktionelle Verknüpfungsreagenzien) oder unterschiedlich sind (heterobifunktionelle Verknüpfungsreagenzien). Die funktionellen Gruppen reagieren mit entsprechenden reaktiven Aminosäureseitenketten von Proteinen und bilden somit zwischen den Seitenketten Brücken aus. Die Brückenbildung kann entweder intramolekular erfolgen und damit beispielsweise genutzt werden, um Abstände zwischen zwei eng benachbarten, reaktiven Gruppen im Protein zu bestimmen, wie z.B. im aktiven Zentrum von Enzymen; oder die Brückenbildung erfolgt intermolekular zwischen zwei oder mehreren Proteinmolekülen, so daß sich höhermolekulare Komplexe ausbilden. Solche intermolekularen Verknüpfungsreagenzien können beispielsweise zur Aufklärung von Antigen-Antikörper-Interaktionen, des Aufbaus von Multienzymkomplexen, der Anordnung von Proteinen in Membranen u.v.m. genutzt werden. Neben nichtspaltbaren bifunktionellen Verknüpfungsreagenzien werden vermehrt auch spaltbare Verknüpfungsreagenzien eingesetzt.

Zu den gegenüber SH-Gruppen reaktiven Verknüpfungsreagenzien gehören u.a. bismaleinimidosubstituierte aliphatische und aromatische Verbindungen, wie beispielsweise die nichtspaltbaren Verbindungen Dimaleinimidohexan, (M.,D. Partis et al. (1983), J. Prot. Chem. 2, 263), 4,4'-Dimaleinimidostilben (P. Chantler, S.M. Bower (1988), J. Biol. Chem. 263, 938) und p-Phenylendimaleinimid (J.E. Moore, W.H. Ward (1956), J. Am. Chem. Soc. 78, 2414), das durch Basen spaltbare Maleinimidomethyl-3-maleinimido-propionat (S. Sato, M. Nakao (1981), J. Biochem. 90, 1177) und einige durch Säuren spaltbare Bis(maleinimidoalkoxy)-Verbindungen (K. Srinivasachar, D.M. Jr. Neville (1989), Biochemistry 28, 2501).

Nachteilig bei den bisher genutzten, mit SH-Gruppen reagierenden, spaltbaren Bismaleinimido-Derivaten ist, daß die nachträgliche Spaltung der Verknüpfungsreagenzien von den Proteinen in sauren oder alkalischen Medien erfolgen muß; diese Reaktionsbedingungen können zur Denaturierung der Proteine führen, so daß deren funktioneller Nachweis nach der Spaltungsreaktion nicht mehr möglich ist. Auch treten bei der Spaltungsreaktion bzw. bei der sauren oder basischen Hydrolyse häufig unerwünschte Nebenreaktionen auf.

Es ist daher Aufgabe der Erfindung, neue, mit SH-Gruppen reagierende, spaltbare Verknüpfungsreagenzien bereitzustellen, die die genannten Nachteile nicht mehr aufweisen. Es ist ferner Aufgabe der Erfindung, ein Verfahren zur Herstellung solcher Reagenzien bereitzustellen.

Die der Erfindung zugrunde liegende Aufgabe wird mit dimaleinimi-dosubstituierten Dihydroxyalkanen der allgemeinen Formel 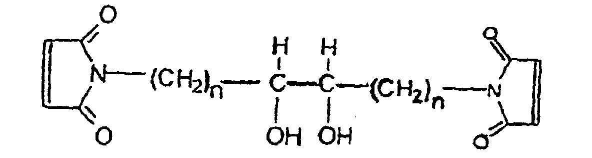 in der n = 1 bis 6, vorzugsweise 1 oder 2, bedeutet, gelöst.

Nach Inkubation von Proteinen mit diesen Verbindungen zeigte sich deren Eignung als homobifunktionelle Verknüpfungsreagenzien. Diese können nach der Verknüpfungsreaktion vom Proteinkomplex wieder gespalten werden, wobei die Spaltungsreaktion durch Periodat-Behandlung unter milden Bedingungen und spezifisch erfolgt. Die Aktivität der Proteine wird durch die oxidative Spaltung nicht beein-flußt, so daß die Spaltbarkeit unter den genannten Bedingungen auch die analytische Identifikation der Verknüpfungspartner ermöglicht.

Die erfindungsgemäße Herstellung der maleinimidosubstituierten Di-hydroxyalkane erfolgt durch Umsetzung der entsprechenden Diamino-alkandiole, insbesondere ihrer Ammoniumsalze, mit N-substituierten Maleinimidoderivaten. Beispielsweise lassen sich die Dihydrohalogenide von Diaminoalkan-diolen mit N-Alkoxycarbonyl-, N-Aryloxycarbonyl- oder N-Hetaryl-oxycarbonyl-maleinimid in Tetrahydrofuran und Wasser in Gegenwart einer Base, wie NaHCO₃, in die entsprechenden, erfindungsgemäßen Verbindungen überführen.

Nachfolgend wird die Erfindung anhand von Ausführungsbeispielen näher erläutert.

Es zeigen:
- Figur 1: Vernetzung und oxidative Spaltung von Na/Ca, K-Austauscher aus Rinderretinen;
- Figur 2: Oxidative Spaltung der Protein-Verbrückung in der Gelmatrix

### Ausführungsbeispiele

### 1. Verfahren zur Herstellung von D,L-Dimaleinimido-2,3-butandiol

1,0 g (12 mmol) NaHCO₃ werden in einer Mischung aus 15 ml Tetra-hydrofuran und 6 ml Wasser auf 0° C gekühlt und unter schnellem Rühren mit 846 mg (3 mmol) D,L-1,4-Diamino-2,3-butandiol-dihydro-bromid (P.W. Feit, O.T. Nielsen (1967), J. Med. Chem. 10, 697) versetzt. Es wird 2 Minuten gerührt, und anschließend werden portionsweise 1,117 g (7,2 mmol) N-Methoxycarbonylmaleinimid hin-zugefügt. Nachfolgend wird 3 Stunden bei Raumtemperatur gerührt, mit etwas Wasser versetzt und mehrmals mit Essigsäureethylester ausgeschüttelt. Die Essigsäureethylesterextrakte werden mit Wasser und NaCl-Lösung ausgezogen, über MgSO₄ getrocknet und am Rotationsverdampfer eingeengt. Das als farbloser Feststoff ausgefallene D,L-1,4-Dimaleinimido-2,3-butandiol wird abgesaugt und mit Ether und Pentan gewaschen.
Ausbeute: 164 mg (19,5 % der Theorie)
Schmelzbereich: 202-204° C (Zersetzung)

| | | | | |
|---|---|---|---|---|
| C₁₂H₁₂O₆N₂ (280,24) | berechnet C | 51,43 H | 4,32 | N 10,0 |
| | gefunden C | 51,64 H | 4,51 | N 9,63 |

### 2. Verwendung von D,L-1,4-Dimaleinimido-2,3-butandiol als spaltbares homobifunktionelles Verknüpfungsreagenz

Die Eignung des dimaleinimidosubstituierten Dihydroxylkans als Verknüpfungsreagenz wurde am Beispiel der Na/Ca,K-Austauscher der Wirbeltierretina nachgewiesen. Diese Proteine mit einer apparenten Molmasse von 240 kD/mol bilden nach Verknüpfung mit den SH-spezifischen Reagenzien p- und o-Phenylendimaleinimid nichtspaltbare Verknüpfungsprodukte der Molmasse 490 kD/mol. Diese sind nach reduktiver elektrophoretischer Auftrennung und Elektroelution auf eine proteinimmobilisierende Membran (PVDF-Membran, Millipore) immunochemisch nachweisbar (P.J. Bauer (1995), Biophysical J. 68, A19).

Dasselbe Verknüpfungsprodukt wurde auch nach Inkubation in 50 µM D,L-1,4-Dimaleinimido-2,3-butandiol für 10 Minuten bei Raumtemperatur erhalten. Die Verknüpfung des Proteins konnte mit 15 mM Natriumperiodat in 20 Minuten bei Raumtemperatur und einem pH-Wert von 7,5 oxidativ gespalten werden. Ein Verknüpfungsprodukt war danach nicht mehr nachweisbar.

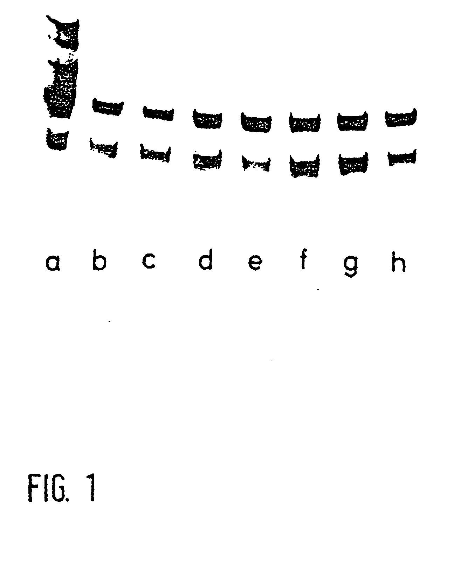

Figur 1 zeigt entsprechend den Nachweis der Vernetzung von Na/Ca,K-Austauschern aus Rinderretinen mit dem dimaleinimido-substituierten Dihydroxyalkan und der oxidativen Spaltung nach Einwirkung von Natriumperiodat. Der Nachweis erfolgte mittels SDS-Elektrophorese im 3,5 % bis 7,5 % Polyacrylamid-Gradientengel mit anschließender Elektroelution auf eine PVDF-Immobilon Membran (Millipore). Das Protein wurde immunochemisch mit dem monoklonalen Antikörper PMex2D9 markiert. Die Vernetzung erfolgte in 100 µM D,L-1,4-Dimaleinimido-2,3-butandiol. Es zeigen
a: vernetztes Protein ohne anschließende Spaltung (markiert wird auch ein proteolytisches Abbauprodukt bei 160 kD)
b: nicht-vernetzte Probe
c-h: vernetzte Proben, die vor der Elektrophorese unterschiedlich oxidativ gespalten wurden, und zwar
   c: 50 Minuten in 50 mM NaIO₄
   d: 50 Minuten in 30 mM NaIO₄
   e: 50 Minuten in 15 mM NaIO₄
   f: 20 Minuten in 50 mM NaIO₄
   g: 20 Minuten in 30 mM NaIO₄
   h: 20 Minuten in 15 mM NaIO₄

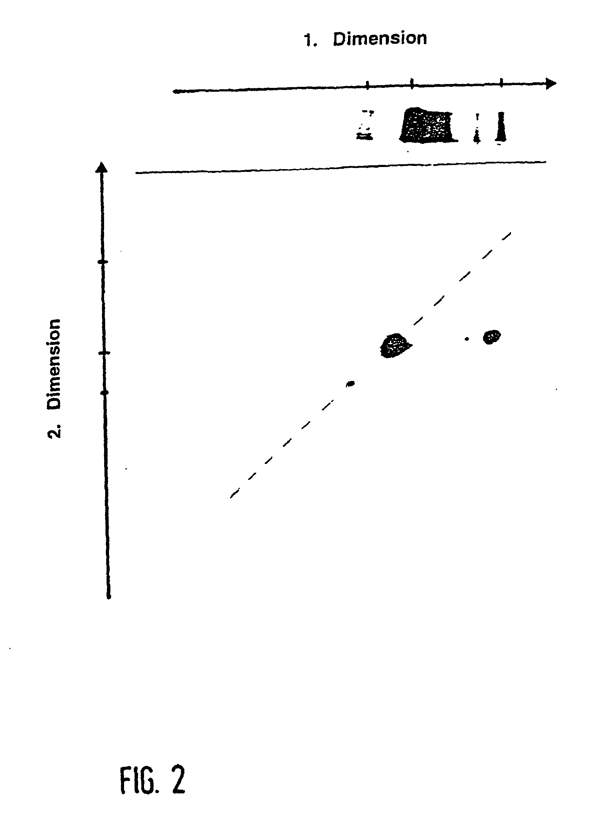

Wie in Figur 2 dargestellt, ließ sich das Verknüpfungsprodukt auch in Polyacrylamidgel (3,5 % bis 7,5 %) mit 30 mM Natriumperiodat in 30 Minuten bei Raumtemperatur oxidativ spalten. Zuvor erfolgte die Proteinvernetzung durch Inkubation in 50 µM D,L-1,4-Dimaleinimido-2,3-butandiol für 10 Minuten. Anschließend wurde die Probe durch SDS-Elektrophorese in einem 3,5 % - 7,5 % Polyacrylamid-Gradientengel aufgetrennt. Der Immunoblot mit PMex2D9-Markierung ist in der Figur 2 oben (1. Dimension) gezeigt. Vor der oxidativen Spaltung mußte der Laufpuffer Tris(hydroxymethyl)aminomethan durch Triethanolamin ersetzt werden, da Tris(hydroxymethyl)aminomethan mit Natriumperiodat reagiert. Eine zweite Gelspur wurde ausgeschnitten und der Laufpuffer gegen 20 mM Triethanolamin-HCl pH 7,5 bei Raumtemperatur ausgetauscht (Inkubation 2 Stunden). Anschliessend wurde die Verbrückung mit 30 mM Natriumperiodat bei Raumtemperatur gespalten (30 Minuten). Die zweite SDS-Elekrophorese (2. Dimension) erfolgte wiederum in einem 3,5 % - 7,5 % Polyacryl-amid-Gradientengel. Die in Figur 2 eingezeichneten Achsen geben die Richtung aufsteigender Molmassen an, wobei die Molmassen 165 kD, 240 kD und 490 kD auf beiden Achsen eingezeichnet wurden. Gespaltene Vernetzungsprodukte müssen unterhalb der gestrichelt gezeichneten Diagonalen auftreten, während nicht-gespaltene Proteine auf der Diagonalen zu liegen kommen. Der Immunoblot dieses Gels zeigt, daß bei 490 kD kein Vernetzungsprodukt mehr zu erkennen ist und die oxidative Spaltung wieder vollständig zum 240 kD Ausgangsprotein zurückführte.

## Patentansprüche

1. Dimaleinimidosubstituierte Dihydroxyalkane der allgemeinen Formel 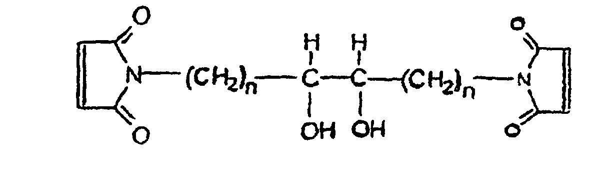 in der n = 1 bis 6 bedeutet.

2. Dimaleinimidosubstituierte Dihydroxyalkane der allgemeinen Formel gemäß Anspruch 1, wobei n 1 oder 2 bedeutet.

3. Verfahren zur Herstellung von dimaleinimidosubstituierten Dihydroxyalkanen der allgemeinen Formel gemäß Anspruch 1, bei dem ein entsprechendes Diaminoalkandiol mit einem N-substituierten Maleinimidoderivat umgesetzt wird.

4. Verfahren nach Anspruch 3,
**dadurch gekennzeichnet,**
**daß** für die Umsetzung Ammoniumsalze des entsprechenden Diaminoalkandiols eingesetzt werden.

5. Verfahren nach Anspruch 3 oder 4,
**dadurch gekennzeichnet,**
**daß** als N-substituiertes Maleinimidoderivat N-Alkoxy-carbonyl-, N-Aryloxycarbonyl- oder N-Hetaryloxycarbonyl-maleinimid eingesetzt wird.

6. Verwendung von dimaleinimidosubstituierten Dihydroxyalkanen der allgemeinen Formel gemäß Anspruch 1 als Verknüpfungsreagenzien.

## Revendications

1. Dihydroxyalcanes à substitution dimaléinimido de formule générale 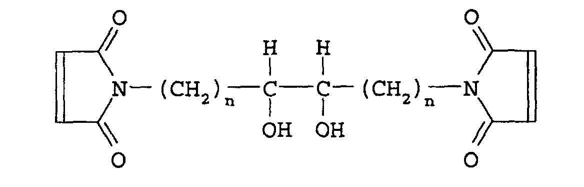 dans laquelle n représente de 1 à 6.

2. Dihydroxyalcanes à substitution dimaléinimido de formule générale selon la revendication 1, dans laquelle n représente 1 ou 2.

3. Procédé de préparation de dihydroxyalcanes à substitution dimaléinimido de formule générale selon la revendication 1, dans lequel un diaminoalcanediol correspondant est mis à réagir avec un dérivé maléinimido N-substitué.

4. Procédé selon la revendication 3, **caractérisé en ce que** l'on utilise pour la réaction des sels d'ammonium du diaminoalcanediol correspondant.

5. Procédé selon la revendication 3 ou la revendication 4, **caractérisé en ce que** l'on utilise comme dérivé maléinimido N-substitué un maléinimide d'alcoxycarbonyle, N-aryloxycarbonyle ou N-hétéroaryloxycarbonyle.

6. Utilisation de dihydroxyalcanes à substitution dimaléinimido de formule générale selon la revendication 1 comme réactifs de réticulation.

## Claims

1. Dimaleinimido-substituted dihydroxyalkanes having the general formula 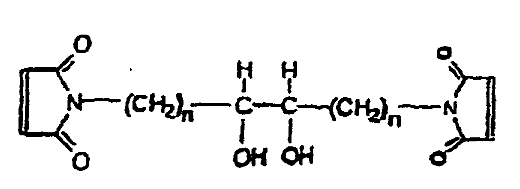 wherein n denotes 1 to 6.

2. Dimaleinimido-substituted dihydroxyalkanes having the general formula according to claim 1, wherein n denotes 1 or 2.

3. Process for the preparation of dimaleinimido-substituted dihydroxyalkanes having the general formula according to claim 1, wherein a corresponding diaminoalkanediol is reacted with an N-substituted maleinimido derivative.

4. Process according to claim 3,
**characterised in that**
ammonium salts of the corresponding diaminoalkanediol are used for the reaction.

5. Process according to claim 3 or 4,
**characterised in that**
N-alkoxycarbonyl, N-aryloxycarbonyl or N-hetaryloxycarbonyl maleinimide is used as the N-substituted maleinimido derivative.

6. Use of dimaleinimido-substituted dihydroxyalkanes having the general formula according to claim 1 as crosslinking reagents.
